# FASCICULE DE BREVET EUROPEEN

(11) **EP 0 800 386 B1**
(45) Date de publication et mention de la délivrance du brevet: **11.11.1998**
(21) Numéro de dépôt: 95943245.1
(22) Date de dépôt: 19.12.1995
(51) Int. Cl.: A61K 9/70

(54) **SYSTEME TRANSDERMIQUE D'ADMINISTRATION SIMULTANEE DE PLUSIEURS PRINCIPES ACTIFS**
TRANSDERMALES SYSTEM ZUR GLEICHZEITIGEN VERABREICHUNG VON MEHREREN WIRKSTOFFEN
TRANSDERMAL SYSTEM FOR THE SIMULTANEOUS DELIVERY OF A NUMBER OF ACTIVE PRINCIPLES

(30) Priorité: 21.12.1994 FR 9415416
(43) Date de publication de la demande: 15.10.1997
(73) Titulaire: LABORATOIRES D'HYGIENE ET DE DIETETIQUE, 75008 Paris (FR)
(72) Inventeur: BEVAN, Bruno, F-21800 Chevigny-Saint-Sauveur (FR); AILLAUD, Cécile, F-21240 Talant (FR)
(74) Mandataire: Clisci, Serge
(86) Numéro de dépôt international: FR9501696
(87) Numéro de publication internationale: WO9619203

(56) Documents cités:
- EP-A- 0 288 734
- WO-A-90/06736
- WO-A-94/06383
- US-A- 4 666 441

## Description

### Domaine de l'invention

La présente invention se rapporte à des systèmes permettant l'administration transcutanée de plusieurs principes actifs simultanément, conçus de façon à faciliter l'ajustement des doses administrées pour un ou plusieurs principes actifs et à réduire la surface de tels systèmes afin d'améliorer la sécurité et le confort durant l'utilisation par le patient.

### Art antérieur

Il existe désormais de nombreux dispositifs d'administration par voie percutanée d'un principe actif.

La composition de ces dispositifs est définie dans le but (i) d'assurer une bonne stabilité physico-chimique du principe actif dans le temps, et (ii) d'obtenir un flux d'absorption percutanée optimal par unité de surface. De ce fait la dose de principe actif administrée au cours d'un traitement est principalement déterminée par la surface du dispositif appliqué sur la peau.

Or cette surface ne doit pas être trop importante pour éviter de provoquer une gêne physique lors de l'utilisation et de conduire à l'obtention d'un dispositif présentant une taille et un aspect esthétique qui rendrait son usage rédhibitoire. Ce dernier doit aussi présenter de bonnes propriétés d'adhésion et de cohésion qui permettent une application aisée, agréable et discrète lors de son utilisation.

Ainsi on réalise aujourd'hui des dispositifs contenant un seul principe actif remplissant ces exigences, c'est à dire efficaces, de petites tailles et confortables, sans fluage ni décollement lors de leur utilisation.

En revanche la réalisation de systèmes aussi performants d'administration de deux principes actifs, voire de plus de deux principes actifs, pose encore de nombreux problèmes de plus en plus difficiles à résoudre quand le nombre de principes actifs à administrer augmente.

Parmi les solutions techniques envisagées dans l'art antérieur, on en connaît une première qui fait appel à des systèmes transdermiques constitués d'un dispositif unique contenant tous les principes actifs en mélange. De tels systèmes sont par exemple décrits dans les documents de brevets EP-A-0 285 563, WO-A-92/07589, WO-A-92/07590 et WO-A-94/06383. Si ces systèmes présentent l'avantage d'être de taille réduite, leur mise au point s'avère généralement très complexe voire irréalisable.

En effet de part leur nature et leurs propriétés physico-chimiques les principes actifs se comportent de façons différentes vis-à-vis de la couche cutanée et ont souvent des influences importantes sur la composition du dispositif.

Les principes actifs présentant des perméabilités cutanées différentes, on a donc des flux d'absorption différents pour chaque principe actif. Il s'avère alors quasi-impossible d'obtenir la dose thérapeutique souhaitée à administrer pour chaque principe actif à partir, à la fois, de la même surface d'absorption et de la même formulation.

De plus, si pour l'un au moins des principes actifs, il est nécessaire de réajuster la dose administrée en cours de développement clinique, il est impossible de modifier la dose administrée de ce principe actif de façon indépendante de celles des autres principes actifs sans devoir changer la formulation des autres composants.

De même il est par ailleurs fréquent qu'un même système d'administration transcutanée de plusieurs principes actifs soit utilisé pour administrer plusieurs dosages de ces principes actifs selon les patients ou les pathologies traités.

Pour cela, différentes surfaces dudit système seront retenues en raison du fait que la dose de principe actif administrée sera proportionnelle à la surface appliquée sur la peau.

Dans le cas d'un tel système d'administration de plusieurs principes actifs et nécessitant différentes posologies, si au moins deux principes actifs ne restent pas dans un même rapport de doses pour toutes les posologies retenues ou si un principe actif reste administré à dose fixe, il sera impossible d'obtenir les différentes posologies souhaitées par la variation de la surface du dispositif car dans ce cas, les doses de chaque principe actif varient simultanément en fonction de la surface et selon des rapports de doses constantes.

Dans les deux cas ci-dessus on perd alors le bénéfice du travail déjà réalisé et on abandonne des systèmes confortables qui présentent de bons flux et de bonnes propriétés physiques.

Or le choix des composants entrant dans la formulation du dispositif se restreint très rapidement avec l'augmentation du nombre de principes actifs car ces derniers imposent souvent des contraintes opposées.

En effet, ces derniers peuvent être partiellement ou totalement incompatibles avec certains constituants de la formulation (résines, solvants, plastifiants, polymères, promoteurs d'absorption cutanée, etc.). Ils peuvent présenter des solubilités et des températures de stabilité différentes et certains d'entre eux recristallisent au cours du temps, se dégradent lors de la mise en oeuvre ou ne sont utilisables dans la composition qu'à des concentrations trop faibles pour obtenir le dosage thérapeutique visé. De même, il n'existe pas de promoteur d'absorption cutanée universel pour tous les principes actifs pour augmenter leurs flux percutanés. Donc, pour administrer des principes actifs différents, il faut souvent utiliser plusieurs promoteurs ou solvants. Or l'introduction de toute nouvelle substance pourra provoquer ou soulever des problèmes d'irritation et de cohésion ou d'adhésion du système.

De la même manière l'ensemble de ces contraintes (compatibilité, solubilité, etc.) joue aussi entre les différents constituants de la formulation autres que les principes actifs et augmente donc les difficultés pour optimiser leur rôle dans la formulation et tirer profit des avantages spécifiques qu'ils peuvent apporter.

En pratique, cette première solution technique n'est pas réalisable et conduit à une impasse ou, dans le meilleur des cas, à des dispositifs qui s'accompagnent d'inconvénients évidents pour l'usage de ce type de forme pharmaceutique.

Une seconde solution connue pour l'administration simultanée de plusieurs principes actifs consiste à réaliser un système formé de plusieurs dispositifs transdermiques appliqués sur la peau contenant chacun un seul principe actif. De tels systèmes sont par exemple décrits dans les documents de brevets WO-A-94/06383, WO-A-90/06736 et WO-A-94/13354.

On évite ainsi les problèmes précédents de compatibilité, stabilité et de réglage du dosage souhaité. L'obtention de la dose recherchée pour chaque principe actif est alors définie par la surface de chaque dispositif.

C'est là que réside le principal inconvénient de cette solution car un tel système présente en général une surface globale importante, d'autant plus grande que le nombre de principes actifs est plus important.

Or, en général, plus un système transdermique a une taille élevée plus il est difficile à utiliser car il devient moins facile d'optimiser ses propriétés d'adhésion et de cohésion sur l'ensemble de la surface qui doit venir en contact avec la peau.

Ainsi plus le système sera grand, plus on risquera d'accentuer les risques de fluage de la masse adhésive donc de souillage des vêtements, les sensations de tiraillement, de gêne voire d'irritation cutanée ou de rupture cohésive lors du retrait, ce qui diminue la maniabilité et l'acceptabilité du système.

D'autre part, la dose administrée au cours du temps étant déterminée par la surface du dispositif appliqué sur la peau, toute augmentation de surface augmente le risque d'un décollement partiel ou total du système ou l'apparition de plis, ce qui peut entraîner une perte de l'activité à cause d'un contact non uniforme sur la peau, en particulier sur des parties courbes du corps ou souvent en mouvement.

De même, dans le cas d'un réservoir, une répartition non homogène sur l'ensemble de la surface devant venir en contact avec la peau entraîne obligatoirement une modification de la dose libérée et ne permet pas d'obtenir l'activité thérapeutique recherchée. Ainsi pour un système réservoir de trop grande taille, pour lequel, sous l'effet de la gravitation, le liquide ou semi-liquide (solution ou gel) contenant le principe actif a tendance à stagner dans la partie inférieure du réservoir, on aura donc une surface utilisée plus réduite et en définitive un système moins efficace.

Un autre désavantage d'un système de grande taille, quelle que soit sa nature, est le risque qu'il soit mal accepté par le patient car trop visible et donc difficile à dissimuler.

L'aspect esthétique et la discrétion du système transdermique éventuellement combinés à une sensation de gêne physique sont en effet des paramètres importants pour l'acceptabilité du produit et le respect du traitément thérapeutique par le patient.

Tous ces problèmes nuisent donc au confort d'utilisation du système ou pire à son efficacité thérapeutique lors de l'utilisation par le patient.

Ainsi les solutions de l'art antérieur ne sont donc pas satisfaisantes car elles ne parviennent pas à concilier la possibilité d'adapter de façon simple les doses administrées de chacun des principes actifs et l'obtention d'un système de surface globale faible permettant une utilisation plus sûre et plus confortable lors de son application sur la peau.

### But de l'invention

Dans le domaine de l'administration simultanée par voie percutanée de plusieurs principes actifs il serait donc souhaitable de disposer d'une nouvelle solution technique permettant de réaliser le compromis recherché sans les inconvénients précités.

C'est ce que propose la présente invention grâce à la réalisation d'un système d'administration simultanée par voie percutanée, d'au moins deux principes actifs, permettant un ajustement simple de la dose à administrer de chaque principe actif tout en présentant une surface totale réduite.

### Objet de l'invention

Le but précité est obtenu grâce à la réalisation, en tant que produit industriel nouveau, d'un nouveau système d'administration par voie percutanée d'au moins deux principes actifs, constitué par la juxtaposition (ou association) d'au moins deux dispositifs, ledit système étant caractérisé en ce qu'il comprend
(i) un premier dispositif qui contient un mélange de l'ensemble des principes actifs dans lequel au moins un premier principe actif (A) est présent selon une quantité permettant d'administrer la dose thérapeutique efficace et au moins un second principe actif (B) est présent selon une quantité inférieure à celle nécessaire pour l'administration de la dose thérapeutique efficace, et
(ii) un ou plusieurs dispositifs supplémentaires contenant chacun un seul principe actif (B) choisi parmi ceux présents dans le premier dispositif selon une quantité inférieure à celle nécessaire pour l'administration de la dose thérapeutique efficace, le ou lesdits dispositifs supplémentaires complétant la quantité de chacun des principes actifs (B) présents dans le système jusqu'à l'obtention de la dose thérapeutique efficace.

### Description détaillée de l'invention

Dans la présente invention l'expression "système transdermique" désigne l'association d'au moins deux dispositifs en vue de réaliser l'administration simultanée de tous les principes actifs par application sur la peau.

Par "dispositif" on entend ici tout ensemble utilisé pour administrer au moins un principe actif par voie percutanée. De tels dispositifs sont généralement classés en deux grandes catégories :
- les dispositifs réservoirs, dans lesquels le ou les principes actifs sont dissous dans un solvant servant de vecteur de transport du principe actif à travers une membrane microporeuse, adhésive ou non adhésive ; et,
- les dispositifs matriciels, dans lesquels le ou les principes actifs sont dissous ou dispersés au sein d'un réseau polymérique constituant la matrice qui peut être auto-adhésive ou non adhésive.

Ces dispositifs peuvent être mono ou multicouches (qualifiés aussi de lamellaires) c'est-à-dire formés par la superposition de plusieurs matrices ou réservoirs qui contiennent ou non un ou plusieurs principes actifs et lesdites matrices ou réservoirs étant éventuellement séparés par des membranes microporeuses.

L'association d'au moins deux dispositifs selon l'invention peut être réalisée suivant des techniques connues de l'homme de l'art comme par exemple contrecollage sur un support adhésivé, enduction double juxtaposée ou thermosoudure des dispositifs sur un support unique. Dans les cas ci-dessus les dispositifs auront un support unique mais on peut aussi fabriquer un système où chaque dispositif a un support indépendant; identique ou différent, l'association ayant lieu par juxtaposition par exemple par thermosoudure des dispositifs ou par association de ces derniers sur un support supplémentaire toujours suivant les mêmes techniques.

Le support utilisé pourra être tout support généralement employé dans les systèmes transdermiques, occlusifs ou non, d'épaisseur variable, qui est imperméable aux constituants des dispositifs.

On préfèrera par exemple un support sous forme de film en polyéthylène, polypropylène, polyester, complexe (ou composite) constitué de polyéthylène et d'un copolymère d'acétate de vinyle et d'éthylène, de film aluminisé ou encore d'une mousse.

De façon pratique l'ensemble du système ou chacun des dispositifs pourra être recouvert d'une couche ou pellicule de protection pelable avant utilisation du système, ledit système pouvant être lui-même emballé dans une protection étanche comme par exemple les complexes polyéthylènealuminium.

Pour réaliser un dispositif selon la présente invention les matières familières à l'homme de l'art peuvent être employées, comme par exemple des polymères naturels ou synthétiques (tels les acryliques ou leurs dérivés, les silicones, les copolymères blocs, les copolymères d'acétate de vinyle et d'éthylène, les caoutchoucs et leurs dérivés etc.), en fonction des propriétés des principes actifs à administrer. On pourra associer à ces derniers d'autres produits appropriés connus, généralement utilisés par l'homme de l'art, comme par exemple des agents de solubilisation, des plastifiants, des résines, des stabilisants, des charges ou des promoteurs de la perméation cutanée.

De même, les éventuelles membranes employées sont celles généralement utilisées dans l'art dans le domaine des systèmes transdermiques comme par exemple un film de copolymère d'éthylène et d'acétate de vinyle.

Dans le cas de l'utilisation dans le système selon la présente invention d'un ou plusieurs dispositifs matriciels; la fabrication de ces derniers est mise en oeuvre suivant les techniques d'enduction généralement connues dans l'art : soit en phase solvant; soit selon la technique dite "hot melt" (c'est à dire en l'absence de solvant).

De même dans le cas de l'utilisation de dispositifs de type réservoir, la fabrication de ces derniers est mise en oeuvre suivant les techniques connues dans l'art comme par exemple création du réservoir par thermoscellage à chaud du support sur une membrane et remplissage simultané ou non du réservoir.

Dans les deux cas, dans le cadre d'une production industrielle, la taille des dispositifs est fixée aux dimensions adaptées, en fonction de la quantité de ou des principes actifs présente par unité de surface, pour obtenir les doses choisies de principes actifs à administrer pendant un temps déterminé par le système.

Le système transdermique selon l'invention peut se présenter sous n'importe quelle forme géométrique, carrée, rectangulaire, circulaire ou ovale. On peut agencer les différents dispositifs soit côte à côte soit concentriquement, chaque dispositif entourant alors complètement le précédent, ou réaliser toute autre construction géométrique. Ils peuvent éventuellement être séparés ou entourés par une ou plusieurs couches supplémentaires qui peuvent être adhésives pour aider si nécessaire au maintien de l'ensemble.

On peut utiliser dans le cadre de la présente invention toute combinaison de principes actifs susceptibles d'être appliqués par voie percutanée qui exercent une action soit topique soit systémique.

Parmi ces combinaisons on peut citer comme possibilités d'associations :
(a) un ou plusieurs oestrogènes avec un ou plusieurs progestatifs, naturels ou synthétiques, dans un but contraceptif ou dans le traitement des symptômes de la ménopause, par exemple l'estradiol, l'éthinylestradiol, l'estriol et leurs dérivés en association avec l'acétate de noréthistérone, le norgestrel, le lévonorgestrel, le désogestrel, le norgestimate, le lynestrénol, le gestodène, l'acétate de nomegestrol ou le diénogest ;
(b) des composés β-bloquants et diurétiques utiles notamment dans les maladies cardio-vasculaires comme par exemple l'association du timolol, pindolol, bufradol, indénolol ou nipradinol avec l'amilonide ou l'hydrochlothiazide ;
(c) des composés corticoïdes et antihistaminiques utiles notamment dans le traitement des allergies comme par exemple l'association de la méthylprednisolone, la prednisolone, l'hydrocortisone, la beclométhazone ou la triamcinolone avec l'astémizole, la dexchlorphéniramine, la cétirizine, le chlorure diphénylhydromine ou la chlorophéniramine ;
(d) des composés antalgiques et anti-inflammatoires utiles notamment dans le traitement de la douleur comme par exemple l'association de l'acide acétylsalicylique, le paracétamol ou la noramidopyrine avec l'acide méfénamique, l'acide flufénamique, le diclofénac, l'oxyphenbutazone, l'ibuprofène, le naxoprène ou le fenbufène ; et,
(e) des composés antibactériens et antibiotiques utiles notamment dans le traitement des infections comme par exemple les associations de l'amoxycilline avec l'acide clavulanique, le sulfamétoxazole avec le triméthoprime, l'érythromycine avec l'acétylsulfafurazole, ou l'érythromycine avec la tétracycline.

De façon avantageuse; on préconise en particulier un système dans lequel les principes actifs à administrer simultanément sont choisis les uns parmi les composés oestrogènes et les autres parmi les composés progestatifs.

### Meilleur mode

Le meilleur mode de mise en oeuvre de l'invention consiste à utiliser un système d'administration par voie percutanée de deux principes actifs (A et B), caractérisé en ce qu'il comprend
(i) un premier dispositif matriciel qui est une matrice contenant dans sa masse le principe actif A selon une quantité permettant d'administrer la dose thérapeutique efficace et le principe actif B selon une quantité inférieure à celle nécessaire pour administrer la dose thérapeutique efficace et
(ii) un second dispositif matriciel qui est une matrice contenant dans sa masse le principe actif B selon une quantité complétant celle présente dans ledit premier dispositif matriciel jusqu'à obtention de sa dose thérapeutique efficace.

En d'autres termes, on préconise un système constitué par la juxtaposition (ou association) de deux matrices, ce système étant tel que, à une première matrice qui contient un mélange des deux principes actifs dans lequel l'un des deux est administré à une dose inférieure à sa dose thérapeutique efficace, on ajoute une seconde matrice, contenant ce même principe actif, qui permet de réaliser l'administration de sa dose thérapeutique efficace.

Ce système est très utile pour l'administration simultanée d'un oestrogène et d'un progestatif, en particulier pour administrer simultanément différentes doses de 17-β-oestradiol comprises entre 25 et 100 µg par 24 heures et différentes doses d'acétate de noréthistérone comprises entre 100 et 800 µg par 24 heures dans des rapports de doses respectives 17-β-oestradiol/acétate de noréthistérone de 1/4 à 1/8, lesdites doses étant thérapeutiquement efficaces dans le traitement des symptômes de la ménopause et des risques cardio-vasculaires qui en découlent.

Les avantages et caractéristiques de l'invention seront mieux compris à la lecture de la description qui va suivre d'exemples de systèmes réalisés selon l'invention et d'essais comparatifs par rapport à des systèmes décrits antérieurement. Bien entendu, l'ensemble de ces éléments n'est nullement limitatif mais est donné à titre d'illustration. Les systèmes selon l'invention et les systèmes comparatifs sont réalisés par différentes combinaisons des dispositifs matriciels décrits ci-après.

### Exemple 1 (dispositif 1)

Dans un récipient on introduit 47,8 g de LEVAPREN® 450P (copolymère d'éthylène et d'acétate de vinyle (en abrégé ci-après : EVA) commercialisé par la société BAYER), 48 g de crotamiton [N-éthyl-2-N-(2-méthylphényl)-2-butenamide](commercialisé par la société BOEHRINGER INGELHEIM), 0,2 g d'IRGANOX® B215 (anti-oxydant commercialisé par la société CIBA-GEIGY) et 115,53 g d'acétate d'éthyle. On chauffe pendant 5 heures jusqu'à dissolution complète de l'EVA. On agite à température ambiante pendant 1 heure puis on ajoute alors 4 g d'acétate de noréthistérone (en abrégé ci-après : NETA) préalablement dissous dans 20 g de tétrahydrofuranne. On agite le mélange obtenu durant environ 30 minutes jusqu'à complète homogénéisation puis on laisse ledit mélange au repos jusqu'à la disparition totale des bulles. On enduit la masse obtenue sur un film de polyester siliconé à température ambiante (15-25°C) de façon à obtenir un dépôt de matière de (100 ± 10) g/m². L'ensemble ainsi obtenu est chauffé à 70° C pendant 30 minutes puis est transféré sur un support polyéthylène. On découpe ensuite le produit résultant selon des dimensions appropriées. Si nécessaire, on conditionne les produits ainsi découpés dans des sachets, enveloppes ou enceintes étanches.

### Exemple 2 (dispositif 2)

On procède de façon analogue à l'exemple 1 ci-dessus, en utilisant 49,8 g de LEVAPREN® 450P, 44 g de crotamiton, 0,2 g d'IRGANOX® B215, 116,2 g d'acétate d'éthyle, 2 g de 17-β-oestradiol et 4 g d'acétate de noréthistérone (introduit en même temps que le 17-β-oestradiol), l'ensemble de ces deux hormones étant dissous dans 30 g de tétrahydrofuranne.

### Exemple 3 (dispositif 3)

Dans un récipient on introduit 20,7 g d'ELVAX® 46L et 6,9 g d'ELVAX® 46 (copolymères d'éthylène et d'acétate de vinyle commercialisés par la société DU PONT), 6 g d'ETHOCEL® (éthylcellulose commercialisée par la société DOW CHEMICAL) sous agitation en chauffant à environ 130° C. On incorpore ensuite progressivement toujours sous agitation et à 130° C, 1,2 g de 17-β-oestradiol et 18,9 g d'EUTANOL® G (2-octyl-dodécanol commercialisé par la société HENKEL) et on agite jusqu'à complète homogénéisation du mélange. On introduit alors à une température de l'ordre de 100 à 110° C, 6,3 g de SURFADONE® LP300 (N-dodécyl-2-pyrrolidone commercialisée par la société GAF CORPORATION) et on maintient l'agitation jusqu'à l'obtention d'un mélange parfaitement homogène. On enduit le mélange, ainsi obtenu, à une température comprise entre 100 et 140° C sur un support intermédiaire provisoire anti-adhérent, notamment un film de polyester siliconé, à raison de (100 ± 10) g/m². On transfère la matrice ainsi obtenue sur un support en polyéthylène.

### Exemple 4 (dispositif 4)

On procède de façon identique à l'exemple 3 mais en utilisant dans ce cas 11 g d'ELVAX® 46L, 11 g d'ELVAX® 46, 5 g d'ETHOCEL®, 15 g d'EUTANOL® G, 5 g de SURFADONE® LP300, 1 g de 17-β-oestradiol et 2 g d'acétate de noréthistérone.

### Exemple 5 (dispositif 5)

On procède de façon identique à l'exemple 3 mais en utilisant dans ce cas 33,75 g d'ELVAX® 46L, 11,25 g d'ELVAX® 46, 10 g d'ETHOCEL®, 30,5 g d'EUTANOL® G, 4 g d'acétate de noréthistérone et 10,5 g de SURFADONE® LP300.

### Exemple 6 (dispositif 6)

Dans un bêcher de 250 ml, on introduit 13,35 g de KRATON G® 1657 (copolymère tribloc poly(styrène-éthylène-butylène-styrène) commercialisé par la société SHELL), 0,1 g d'IRGANOX® 565 (anti-oxydant commercialisé par la société CIBA-GEIGY), 12,5 g de ZONATAC® 105L (résine tackifiante commercialisée par la société ARIZONA CHEMICAL), 10,25 g de PARAPOL® 950 (copolymère de n-butène et d'isobutylène commercialisé par la société EXXON CHEMICAL), 10,25 g d'EUTANOL® G (2-octyl-dodécanol commercialisé par la société HENKEL), 3 g de SURFADONE® LP300 (N-dodécyl-2-pyrrolidone commercialisée par la société GAF CORPORATION) et 25,6 g de cyclohexane. On agite le mélange pendant 6 heures jusqu'à dissolution complète des constituants en chauffant à 60° C. On ajoute alors 0,55 g d'acétate de noréthistérone préalablement dissous dans 2,75 g de tétrahydrofuranne. On agite pendant 30 minutes jusqu'à complète homogénéisation du mélange obtenu qu'on laisse au repos jusqu'à disparition totale des bulles. On enduit le mélange obtenu sur un film de polyester siliconé, à raison de (100 ± 10) g/m² à la température ambiante (15-25° C). On chauffe à 70° C pendant 0,5 heure. On transfère la matrice ainsi obtenue sur un support polyéthylène. On découpe alors à des dimensions appropriées et on conditionne dans des sachets si nécessaire.

### Exemple 7 (dispositif 7)

Dans un bêcher de 250 ml on introduit 13,8 g de VECTOR® 4211D [copolymère tribloc poly(styrène-isoprène-styrène) commercialisé par la société EXXON CHEMICAL], 23,85 g de ECR® 385 (résine tackifiante commercialisée par la société EXXON CHEMICAL), 0,1 g d'IRGANOX® 565 (anti-oxydant commercialisé par la société CIBA-GEIGY), 3,5 g de SURFADONE® LP300 (N-dodécyl-2-pyrrolidone, commercialisée par la société BOEHRINGER INGELHEIM), 7,5 g de LAUROGLYCOL® (mélange de mono- et diester de propylèneglycol et d'acide laurique commercialisé par la société GATTEFOSSE) et 19,8 g d'acétate d'éthyle. On agite ce mélange en chauffant à 60° C jusqu'à dissolution complète des composés. On ajoute alors une solution de 1,25 g d'acétate de noréthistérone préalablement dissous dans 6,25 g de tétrahydrofuranne. On agite le mélange résultant ainsi obtenu, durant environ 30 minutes, jusqu'à complète homogénéisation. On laisse refroidir au repos jusqu'à disparition totale des bulles. On enduit la masse résultante sur un film de polyester siliconé à raison de (110 ± 10) g/m², à température ambiante (15-20° C). L'enduction ainsi réalisée est chauffée à 50° C pendant au moins 30 minutes puis transférée sur un support polyéthylène. On découpe des formes aux dimensions souhaitées.

Afin d'illustrer les avantages de la présente invention on a réalisé des tests de perméation ex-vivo sur peau abdominale de souris "nude" mâles suivant le protocole suivant.

La mesure des quantités d'hormones (i.e. stéroïdes) libérées par un dispositif transdermique d'une surface de 2,54 cm² préalablement découpé à l'emporte pièce et déposé sur un disque de 3,14 cm² de peau abdominale de souris "nude" mâle est effectuée en cellule statique en verre thermostatée à 37° C et ayant un compartiment récepteur d'un volume de 11,5 ml contenant une phase réceptrice formée par un mélange sérum physiologique/PEG₄₀₀ (75/25 ; v/v).

On effectue des prélèvements à 2, 4, 6, 8, 12, 16, 20 et 24 heures dans les solutions réceptrices et on les dose par chromatographie liquide. Compte tenu de la variabilité des résultats liés à la perméabilité intrinsèque des échantillons de peau, chaque essai de perméation pour un échantillon de dispositif transdermique est réalisé sur un nombre minimum de 3 à 5 échantillons de peau. Le résultat donné est la moyenne obtenue pour chaque dispositif à partir de ces essais.

On a ainsi obtenu pour le 17-β-oestradiol (F_{ES}) et/ou l'acétate de noréthistérone (F_{NETA}) les flux d'absorption cutanée moyens suivants dans le cas des dispositifs 1 à 7.
- **Dispositif 1** :: F_{NETA} = 0,35 ± 0,16 µg/cm²/h
- **Dispositif 2** :: F_{ES} = 0,2 ± 0,07 µg/cm²/h
F_{NETA} = 0,39 ± 0,1 µg/cm²/h
- **Dispositif 3** :: F_{ES} = 0,61 ± 0,08 µg/cm²/h
- **Dispositif 4** :: F_{ES} = 0,57 ± 0,13 µg/cm²/h
F_{NETA} = 0,57 ± 0,17 µg/cm²/h
- **Dispositif 5** :: F_{NETA} = 0,5 ± 0,03 µg/cm²/h
- **Dispositif 6** :: F_{NETA} = 0,47 ± 0,05 µg/cm²/h
- **Dispositif 7** :: F_{NETA} = 0,89 ± 0,12 µg/cm²/h

Les tableaux I à V illustrent le gain de surface obtenu par les systèmes selon l'invention par rapport à un système comparatif formé de deux dispositifs juxtaposés contenant chacun un seul principe actif dans le cas de l'administration simultanée par voie percutanée du 17-β-oestradiol et de l'acétate de noréthistérone.

On a ainsi comparé dans le tableau I un système I selon l'invention, constitué des dispositifs 4 et 5, à un système comparatif Ia constitué des dispositifs 3 et 5.

Dans le tableau II on a comparé un système II selon l'invention constitué des dispositifs 2 et 1 à un système comparatif IIa constitué des dispositifs 3 et 1.

Dans le tableau III on a comparé un système III selon l'invention, constitué des dispositifs 4 et 1 à un système comparatif IIIa constitué des dispositifs 3 et 1.

Dans le tableau IV on a comparé un système IV selon l'invention, constitué des dispositifs 4 et 6, à un système comparatif IVa constitué des dispositifs 3 et 6.

Dans le tableau V on a comparé un système V selon l'invention, constitué des dispositifs 4 et 7, à un système comparatif Va constitué des dispositifs 3 et 7.

Dans ces tableaux les abréviations utilisées ont les significations suivantes :
SD₁ représente la surface du premier dispositif exprimée en cm².
SD₂ représente la surface du second dispositif exprimée en cm².
S représente la surface totale du système formé par la juxtaposition des deux dispositifs exprimée en cm².
G représente le gain de surface exprimé en pourcentage pour les systèmes selon l'invention (représentés par la seconde ligne des tableaux) par rapport à des systèmes formés par la juxtaposition de deux dispositifs contenant chacun un seul principe actif (représentés par la première ligne des tableaux).

Le premier dispositif contient un mélange de 17-β-oestradiol et d'acétate de noréthistérone dans le cas des systèmes selon l'invention ou le 17-β-oestradiol seul dans le cas des systèmes comparatifs.
Le second dispositif contient toujours l'acétate de noréthistérone seul.

**Tableau I**

| | **SD**_{**1**} | **SD**_{**2**} | **S** | **G** |
|---|---|---|---|---|
| **Système comparatif Ia** | 3,4 | 20,8 | 24,2 | 16,1 |
| **Système I** | 3,6 | 16,7 | 20,3 | |

**Tableau II**

| | **SD**_{**1**} | **SD**_{**2**} | **S** | **G** |
|---|---|---|---|---|
| **Système comparatif IIa** | 3,4 | 29,8 | 33,2 | 14,1 |
| **Système II** | 10,4 | 18,1 | 28,5 | |

**Tableau III**

| | **SD**_{**1**} | **SD**_{**2**} | **S** | **G** |
|---|---|---|---|---|
| **Système comparatif IIIa** | 3,4 | 29,8 | 33,2 | 17,3 |
| **Système III** | 3,6 | 23,8 | 27,4 | |

**Tableau IV**

| | **SD**_{**1**} | **SD**_{**2**} | **S** | **G** |
|---|---|---|---|---|
| **Système comparatif IVa** | 3,4 | 22,2 | 25,6 | 16,8 |
| **Système IV** | 3,6 | 17,7 | 21,3 | |

**Tableau V**

| | **SD**_{**1**} | **SD**_{**2**} | **S** | **G** |
|---|---|---|---|---|
| **Système comparatif Va** | 3,4 | 11,7 | 15,1 | 13,9 |
| **Système V** | 3,6 | 9,4 | 13 | |

Dans le cas présent on souhaite administrer les doses thérapeutiquement efficaces suivantes :
- 50 µg de 17-β-oestradiol par 24 heures, et
- 250 µg d'acétate de noréthistérone par 24 heures.

Si l'on veut administrer simultanément ces deux hormones contenues au sein d'un dispositif unique il faut que la différence de perméabilité cutanée donc de flux d'absorption cutanée entre l'acétate de noréthistérone et le 17-β-oestradiol soit de 5. Or dans la pratique l'obtention d'une telle différence, théoriquement réalisable bien que déjà difficile en tant que telle à mettre en oeuvre, s'avère impossible si l'on tient compte des contraintes de stabilité, de confort et des propriétés adhésives et cohésives qu'exigent la commercialisation d'un tel dispositif.

Ainsi les dispositifs 2 et 4, qui présentent ces bonnes propriétés physico-chimiques et un bon confort, ne permettent pas d'obtenir l'ajustement des doses recherchées.

Il est impossible d'obtenir la dose efficace souhaitée pour l'acétate de noréthistérone sans multiplier respectivement par 2,5 et 5 les doses de 17-β-oestradiol administrées. La solution alternative, qui utilise un système formé de 2 dispositifs matriciels juxtaposés contenant l'un le 17-β-oestradiol et l'autre l'acétate de noréthistérone, est moins performante que celle selon l'invention qui permet un gain de surface par rapport à cette dernière grâce à l'association d'un premier dispositif matriciel contenant un mélange des deux hormones dans lequel la concentration en acétate de noréthistérone ne permet pas d'obtenir la dose de 250 µg par 24 heures recherchée et d'un second dispositif contenant l'acétate de noréthistérone seul permettant d'apporter la dose complémentaire nécessaire à l'ajustement des 250 µg par 24 heures.

Ainsi dans le tableau I pour un système comparatif Ia, avec le dispositif 3 qui présente un flux d'absorption cutanée de 0,61 µg/cm²/h, pour administrer 50 µg de 17-β-oestradiol en 24 heures, il faut utiliser un dispositif d'une surface de 3,4 cm².

De même pour administrer 250 µg d'acétate de noréthistérone en 24 heures à partir du dispositif 5, qui présente un flux d'absorption cutanée de 0,5 µg/h/cm², il faut utiliser un dispositif d'une surface de 20,8 cm². Soit donc un système comparatif Ia d'une surface globale de 24,2 cm².

Par contre pour un système I selon l'invention on constate qu'avec le dispositif 4, qui présente des flux d'absorption cutanée de 0,57 µg/h/cm² en acétate de noréthistérone et en 17-β-oestradiol, il faut utiliser pour administrer 50 µg de 17-β-oestradiol en 24 heures un dispositif d'une surface de 3,6 cm². Ce dispositif de 3,6 cm² va permettre d'administrer simultanément 49,2 µg d'acétate de noréthistérone en 24 heures. Il reste donc à administrer 200,8 µg d'acétate de noréthistérone pour obtenir la dose de 250 µg recherchée. Cette quantité sera obtenue à l'aide du dispositif 5 qui présentera, pour un flux d'absorption cutanée de 0,5 µg/h/cm², une surface de 16,7 cm².

Le système I complet aura donc une surface de 20,3 cm², soit un gain de 16,1 % par rapport au système comparatif Ia précédemment décrit fondé sur les dispositifs 3 et 5.

De la même façon l'analyse du tableau II montre qu'avec un système II selon l'invention on obtient un gain de surface de 14,1 %. Ce résultat est très intéressant, car dans ce cas le flux (0,2 ± 0,07 µg/h/cm²) en 17-β-oestradiol du dispositif 2 du système II selon l'invention, contenant les deux hormones est beaucoup plus faible que celui du dispositif 3 contenant le 17-β-oestradiol seul (F_{ES} = 0,61 µg/h/cm²) du système comparatif IIa ce qui impose une surface du dispositif 2 dans le système selon l'invention très importante (10,4 cm²). Malgré cela on obtient un gain de surface avec le système II selon l'invention intéressant par rapport au système comparatif IIa. Ce résultat est d'autant plus remarquable que le dispositif 2 présente aussi un flux en acétate de noréthistérone faible de 0,39 µg/h/cm² ce qui entraîne que la quantité d'acétate de noréthistérone à délivrer par le dispositif 1 complémentaire est encore importante.

On peut aussi remarquer que, dans ce cas, le flux en acétate de noréthistérone du dispositif 1 commun aux deux systèmes est faible F_{NETA} = 0,35 µg/h/cm².

On constate néanmoins que sur ce dispositif 1, le système II selon l'invention permet un gain de surface important 18,1 cm² contre 29,8 cm² soit 40% par rapport au système comparatif IIa.

Une des conséquences de cette situation est d'augmenter les possibilités de formulation utilisables car même si elles présentent des flux de perméations cutanées faibles cela n'impose pas obligatoirement l'utilisation de surfaces de taille trop importante.

Dans le tableau V, où à l'inverse du cas précédent le flux en acétate de noréthistérone dans le dispositif 7 commun aux deux systèmes est élevé (0,89 µg/h/cm²), ce qui entraîne pour le système comparatif Va (où chaque dispositif contient une seule hormone) une surface globale faible de 15,1 cm², on constate toujours un gain de surface, de l'ordre de 14 %, pour le système V selon l'invention.

On obtient des résultats du même ordre avec les tableaux III et IV où les gains de surface sont respectivement de 17,3 et 16,8 %.

Ces deux tableaux ainsi que le tableau V soulignent encore un autre avantage de la présente invention; l'utilisation pour la réalisation du système de dispositifs de natures différentes.

On a ainsi couplé au dispositif 4 à base de copolymère d'éthylène et d'acétate de vinyle, soit le dispositif 1 à base du même type de copolymère mais de formulation différente (tableau III), soit le dispositif 6 à base de copolymère blocs poly (styrène-éthylène-butylène-styrène) (tableau IV) ou soit le dispositif 7 à base de copolymère bloc poly(styrène-isoprène-styrène) (tableau V).

L'extension du choix des composés utilisables dans les compositions des dispositifs et la capacité d'utiliser des dispositifs à flux faibles citée précédemment facilitent le réglage des dosages recherchés pour chaque principe actif et augmentent donc de façon considérable le champ des possibilités pour la mise au point et l'adaptation à plusieurs posologies de systèmes d'administration simultanée d'au moins deux principes actifs confortables qui présentent des tailles raisonnables.

Bien évidemment ces gains de surface exemplifiés ici pour des systèmes oestroprogestatifs de l'ordre de 15 à 20 %, peuvent être encore plus avantageux suivant la perméabilité des principes actifs utilisés.

## Revendications

1. Système d'administration par voie percutanée d'au moins deux principes actifs, constitué par la juxtaposition d'au moins deux dispositifs, ledit système étant caractérisé en ce qu'il comprend
(i) un premier dispositif qui contient un mélange de l'ensemble des principes actifs dans lequel au moins un premier principe actif (A) est présent selon une quantité permettant d'administrer la dose thérapeutique efficace et au moins un second principe actif (B) est présent selon une quantité inférieure à celle nécessaire pour l'administration de la dose thérapeutique efficace, et
(ii) un ou plusieurs dispositifs supplémentaires contenant chacun un seul principe actif (B) choisi parmi ceux présents dans le premier dispositif selon une quantité inférieure à celle nécessaire pour l'administration de la dose thérapeutique efficace, le ou lesdits dispositifs supplémentaires complétant la quantité de chacun des principes actifs (B) présents dans le système jusqu'à l'obtention de la dose thérapeutique efficace.

2. Système selon la revendication 1 caractérisé en ce que les dispositifs du système sont du type réservoir ou du type matriciels.

3. Système selon la revendication 2 caractérisé en ce que les dispositifs du système sont monocouches, multicouches ou lamellaires.

4. Système selon l'une des revendications 1 à 3 caractérisé en ce que ledit système présente un support unique pour tous les dispositifs.

5. Système selon l'une des revendications 1 à 3 caractérisé en ce que dans ledit système chaque dispositif possède un support indépendant.

6. Système selon la revendication 1, pour l'administration de deux principes actifs (A et B), caractérisé en ce qu'il comprend
(i) un premier dispositif matriciel qui est une matrice contenant dans sa masse le principe actif A selon une quantité permettant d'administrer la dose thérapeutique efficace et le principe actif B selon une quantité inférieure à celle nécessaire pour administrer la dose thérapeutique efficace, et
(ii) un second dispositif matriciel qui est une matrice contenant dans sa masse le principe actif B selon une quantité complétant celle présente dans ledit premier dispositif matriciel jusqu'à obtention de sa dose thérapeutique efficace.

7. Système selon l'une des revendications 1 à 6 caractérisé en ce que les principes actifs à administrer simultanément sont choisis les uns parmi les composés oestrogènes et les autres parmi les composés progestatifs.

8. Système selon la revendication 7 caractérisé en ce qu'il comprend un composé oestrogène qui est le 17-β-oestradiol et un composé progestatif qui est l'acétate de noréthistérone.

## Claims

1. A system for the transdermal delivery of at least two active principles, consisting of at least two juxtaposed devices, said system being characterized in that it comprises
(i) a first device containing a mixture of all the active principles in which at least one first active principle (A) is present in an amount which enables the effective therapeutic dose to be delivered, and at least one second active principle (B) is present in an amount which is less than that required to deliver the effective therapeutic dose, and
(ii) one or more additional devices each containing a single active principle (B) selected from those present in the first device and provided in an amount which is less than that required to deliver the effective therapeutic dose, said additional device or devices making up the amount of each of the active principles (B) present in the system until the effective therapeutic dose is obtained.

2. A system according to claim 1, characterized in that the devices of the system are of the reservoir type or the matrix type.

3. A system according to claim 2, characterized in that the devices of the system are monolayer, multilayer or lamellar.

4. A system according to one of claims 1 to 3, characterized in that said system has a single support for all the devices.

5. A system according to one of claims 1 to 3, characterized in that each device in said system has an independent support.

6. A system according to claim 1 for the delivery of two active principles (A and B), characterized in that it comprises
(i) a first matrix device which is a matrix containing in its bulk the active principle A in an amount which enables the effective therapeutic dose to be delivered, and the active principle B in an amount which is less than that necessary to deliver the effective therapeutic dose, and
(ii) a second matrix device which is a matrix containing in its bulk the active principle B in an amount which makes up the amount present in said first matrix device until its effective therapeutic dose is obtained.

7. A system according to one of claims 1 to 6, characterized in that the active principles to be delivered simultaneously are selected on the one hand from estrogen compounds and on the other hand from progestin compounds.

8. A system according to claim 7, characterized in that it comprises the estrogen compound 17-β-estradiol and the progestin compound norethisterone acetate.

## Patentansprüche

1. System zur perkutanen Verabreichung von mindestens zwei Wirkstoffen, das aus der Anordnung von mindestens zwei Vorrichtungen nebeneinander besteht, wobei das System dadurch gekennzeichnet ist, daß es umfaßt:
i) eine erste Vorrichtung, die eine Mischung aller Wirkstoffe enthält, in welcher mindestens ein erster Wirkstoff (A) in einer Menge vorliegt, die die Verabreichung der therapeutischen Wirkdosis gestattet, und mindestens ein zweiter Wirkstoff (B) in einer Menge vorliegt, die kleiner als die zur Verabreichung der therapeutischen Wirkdosis erforderliche Menge ist, und
ii) eine oder mehrere zusätzliche Vorrichtungen, die jeweils einen einzigen Wirkstoff (B) enthalten, der aus denjenigen, die in der ersten Vorrichtung vorliegen, ausgewählt ist, und zwar in einer Menge, die kleiner als die für die Verabreichung der therapeutischen Wirkdosis erforderliche Menge ist, wobei die zusätzliche/n Vorrichtung/en die Menge jedes der im System vorliegenden Wirkstoffe (B) bis zum Erreichen der therapeutischen Wirkdosis ergänzt/ergänzen.

2. System nach Anspruch 1, dadurch gekennzeichnet, daß die Vorrichtungen des Systems vom Behältertyp oder vom Matrixtyp sind.

3. System nach Anspruch 2, dadurch gekennzeichnet, daß die Vorrichtungen des Systems einschichtig, mehrschichtig oder lamellar sind.

4. System nach einem der Ansprüche 1 bis 3, dadurch gekennzeichnet, daß das System einen einzigen Träger für alle Vorrichtungen aufweist.

5. System nach einem der Ansprüche 1 bis 3, dadurch gekennzeichnet, daß in dem System jede Vorrichtung einen unabhängigen Träger besitzt.

6. System nach Anspruch 1 für die Verabreichung von zwei Wirkstoffen (A und B), dadurch gekennzeichnet, daß es umfaßt:
i) eine erste Matrixvorrichtung, die eine Matrix ist, die in ihrer Masse den Wirkstoff A in einer Menge enthält, die die Verabreichung der therapeutischen Wirkdosis gestattet, und den Wirkstoff B in einer Menge, die kleiner als die zur Verabreichung der therapeutischen Wirkdosis erforderliche Menge ist, und
ii) eine zweite Matrixvorrichtung, die eine Matrix ist, die in ihrer Masse den Wirkstoff B in einer Menge enthält, die die in der ersten Matrixvorrichtung vorliegende Menge bis zum Erreichen ihrer therapeutischen Wirkdosis ergänzt.

7. System nach einem der Ansprüche 1 bis 6, dadurch gekennzeichnet, daß die gleichzeitig zu verabreichenden Wirkstoffe zum einen aus den Estrogenen und zum anderen aus den Gestagenen ausgewählt sind.

8. System nach Anspruch 7, dadurch gekennzeichnet, daß es ein Estrogen enthält, nämlich 17-β-Estradiol, sowie ein Gestagen, nämlich Norethisteronacetat.
